# EUROPEAN PATENT APPLICATION

(11) **EP 3 159 400 A1**
(43) Date of publication of application: **26.04.2017**
(21) Application number: 15810868.8
(22) Date of filing: 23.06.2015
(51) Int. Cl.: C12M 1/42, B03C 5/00

(54) **CELL LOCATION UNIT, ARRAY, DEVICE AND FORMATION METHOD THEREOF**

(30) Priority: 23.06.2014 CN 201410284161
(71) Applicant: Ocular Fluidics, Inc., Moraga, CA 94556 (US)
(72) Inventor: HUANG, Chengjun, Beijing 100029 (CN); LUO, Jun, Beijing 100029 (CN); ZHAO, Chao, Beijing 100029 (CN)
(74) Representative: Hally, Anna-Louise
(86) International application number: PCT/CN2015/082034
(87) International publication number: WO 2015/196957

(57) **Abstract**

The present disclosure provides a cell positioning unit, array, device and a method for manufacturing the same. The cell positioning unit comprises: a substrate; at least a pair of microelectrodes on the substrate, wherein the microelectrodes are disposed at intervals on a circumference such that pDEP force are generated in an area where tips of the microelectrodes aggregate, and wherein a voltage signal applied to at least one microelectrode has a phase difference with a voltage signal applied to another microelectrode; and a cell positioning hole on the microelectrodes, wherein the cell positioning hole has an accommodation space which exposes the pDEP force fields and accommodates a single cell. By applying an AC voltage signal having a certain amplitude, frequency and phase to the microelectrodes, cells will move to surfaces of the microelectrodes, such that a single cell can be accurately positioned to a certain place by means of the cell positioning hole and the microelectrodes.

## Description

The present disclosure claims priority of Chinese Patent Application No. 201410284161.8 entitled "Cell Location Unit, Array, Device and Formation Method Thereof" filed on June 23, 2014, the entirety of which is incorporated herein by reference.

### Technical Field

The present disclosure relates to cell detection, and in particular, to a cell positioning unit, array, device and a method for manufacturing the same.

### Background

Cells are the fundamental units of life. In the field of life science and medical science, it is necessary to analyze and study cells. The manipulation and analysis of a single cell is one of the research interests which have drawn much attention currently.

Microfluidic chip is developed based on the Micro-Electro-Mechanical Systems (MEMS) technique, and widely applied in cell manipulation and detection, for example, operation, transportation and reaction of cell population or a single cell, because of its advantages including small volume, high integration degree and fast response speed.

In addition, the application of Dielectrophoresis (DEP) in cell manipulation, separation and detection has become one of the research focuses. Dielectrophoresis refers to an electrokinetic movement arising on a dielectrical object, such as biological cells or other particles, subjected to a non-uniform electric field caused by polarization effects. DEP can be classified as positive DEP (pDEP) and negative DEP (nDEP) according to the translational directions of cells. By means of cell Dielectrophoresis, manipulation and selective separation of cell population can be achieved, and furthermore, single cell positioning, detecting, etc. can also be obtained.

Cell positioning by pDEP or nDEP has been reported in related art such as literature and published patents/applications. Generally, in cell positioning by nDEP, a "DEP trap" is produced in the symmetric center of a group of positioning microelectrodes. The DEP forces on cells in the DEP trap are in an equilibrium condition. However, cells are driven by nDEP to places having the weakest electric field, such that the DEP force on the cells decreases with the increasing of the distance from the electrode. Therefore, positioning may be restricted due to the weak force for positioning the cells.

Better positioning effects can be achieved by positioning cells with pDEP so as to attract cells to microelectrodes. However, because pDEP may be applied to all the cells near the electrodes such that these cells are attracted to surfaces of the electrode, it cannot be assured that there is only one single cell on each positioning electrode or electrode group, which may lead to limitations.

### Summary of the Disclosure

The present disclosure provides a cell positioning unit, array and device for accurate single cell positioning to address the technical issue described above.

The present disclosure provides a cell positioning unit comprising:
a substrate;
at least a pair of microelectrodes on the substrate, wherein the microelectrodes are disposed at intervals on a circumference such that pDEP force fields are generated in an area where tips of the microelectrodes aggregate, and wherein a voltage signal applied to at least one microelectrode has a phase difference with a voltage signal applied to another microelectrode; and
a cell positioning hole on the microelectrodes, wherein the cell positioning hole has an accommodation space which exposes the area of pDEP force and accommodates a single cell.

Optionally, the microelectrodes have a stripe shape, and the tips of each pair of microelectrodes are disposed oppositely and exposed in the accommodation space of the cell positioning hole.

Optionally, the cell positioning hole has a circular or square shape, and has a diameter or side length of about 1-2 times the diameters of the cell to be detected.

The present disclosure further provides a cell positioning unit comprising:
a substrate;
a pair of annular microelectrodes on the substrate, wherein the pair of microelectrodes are concentrically disposed, and the voltage signals applied to the pair of microelectrodes have a phase difference therebetween; and
a cell positioning hole on the microelectrodes, wherein the cell positioning hole has an accommodation space which exposes the annular microelectrodes and accommodates a single cell.

Optionally, the cell positioning hole and the annular microelectrodes are concentrically disposed, and the cell positioning hole has a circular or square shape and has a diameter or side length of about 1-2 times the diameter of the cell to be detected.

Further, the present disclosure provides a cell positioning array comprising a plurality of cell positioning units described above.

Further, the present disclosure provides a cell positioning device comprising: the cell positioning array described above; and a fluidic chamber on the substrate for accommodating the cell positioning array, wherein a microfluidic chip is disposed in an opening of the fluidic chamber.

Further, the present disclosure provides a method for manufacturing a cell positioning unit/array comprising:
providing a substrate;
forming at least a pair of microelectrodes on the substrate, wherein the microelectrodes are disposed at intervals on a circumference such that pDEP force fields are generated in an area where tips of the microelectrodes aggregate, and wherein a voltage signal applied to at least one microelectrode has a phase difference with a voltage signal applied to another microelectrode; and
forming a cell positioning hole on the microelectrodes, wherein the cell positioning hole has an accommodation space which exposes the pDEP force fields and accommodates a single cell.

Optionally, the microelectrodes have a stripe shape, and wherein tips of each pair of microelectrodes are disposed oppositely and exposed in the accommodation space of the cell positioning hole.

Optionally, the cell positioning hole has a circular or square shape, and has a diameter or side length of about 1-2 times the diameter of the cell to be detected

Furthermore, the present disclosure provides a method for manufacturing a cell positioning unit/array comprising:
providing a substrate;
forming at least a pair of annular microelectrodes on the substrate, wherein the pair of microelectrodes are concentrically disposed, and the voltage signals applied to the pair of microelectrodes have a phase difference therebetween; and
forming a cell positioning hole on the annular microelectrodes, wherein the cell positioning hole has an accommodation space which exposes the annular microelectrodes and accommodates a single cell.

Optionally, the cell positioning hole and the annular microelectrodes are concentrically disposed, and the cell positioning hole has a circular or square shape and has a diameter or side length of about 1-2 times the diameter of the cell to be detected.

Further, the present disclosure provides a method for forming a cell positioning device comprising:
forming a cell positioning array described above;
forming a fluidic chamber on the substrate for accommodating the cell positioning array, wherein a microfluidic chip is disposed in an opening of the fluidic chamber.

Optionally, the fluidic chamber is formed of polymer, and wherein one end of the fluidic chamber is bonded to the substrate while the other end is bonded to the microfluidic chip by an external pressure.

The present disclosure provides a cell positioning unit, array, device and a method for manufacturing the same. By pDEP technique and formation of cell positioning holes on the microelectrodes, a single cell can be positioned in a cell positioning hole to achieve single cell positioning with high accuracy and high-sensitivity. A single cell array structure formed by the present disclosure can greatly improve single cell positioning accuracy and throughput. The single cell which has been positioned can be widely used in single cell analysis and high throughput screening of drugs, etc.

### Brief Description of Drawings

The above and other objects, features, and advantages of the present disclosure will become apparent from following descriptions of embodiments with reference to the attached drawings, in which:
Figure 1 schematically illustrates a structural top view of a cell positioning unit according to a first embodiment of the present disclosure;
Figure 2 schematically illustrates a structural cross-sectional view of a cell positioning unit according to the first embodiment of the present disclosure;
Figure 3 and Figure 4 schematically illustrate the electric field distribution (E) and DEP force factor (∇| E|²) according to a cell positioning unit of the first embodiment of the disclosure, respectively;
Figure 5 schematically illustrates a structural cross-sectional view of a cell positioning unit according to the embodiment of the present disclosure; and
Figure 6 schematically illustrates a structural top view of a cell positioning unit according to a second embodiment of the present disclosure.

### Detailed Embodiments

Hereinafter, descriptions are given for embodiments of the present disclosure with reference to the attached drawings. The same or similar reference numbers denote the same or similar elements or elements having the same or similar function throughout the drawings. Embodiments described with reference to the drawings are illustrative only, and are intended to interpret the disclosure rather than limiting the disclosure.

More details of the present disclosure will be described in the following with reference to embodiments in order to better understand the technical proposal and advantages of the present disclosure.

### First Embodiment

The present disclosure provides a cell positioning unit for single cell positioning by the DEP technique. The cell positioning unit comprises: a substrate; at least a pair of microelectrodes on the substrate disposed at intervals on a circumference to generate pDEP force fields in a region where tips of the microelectrodes aggregate, wherein a voltage signal which is applied to at least one microelectrode has a phase difference with a voltage signal applied to another microelectrode; and a cell positioning hole on the microelectrode, wherein the cell positioning hole has an accommodation space which exposes the pDEP force fields and accommodates a single cell.

In the embodiment, the microelectrodes are disposed at intervals on a circumference and the tips of the microelectrodes are arranged towards an aggregation center. Thus, pDEP force fields are generated in the area where the tips of the microelectrodes aggregate. Alternating current (AC) voltage signals having certain amplitude and frequency are applied to the microelectrodes so that a strongest electric field may be generated in the edge of the microelectrode tips due to the phase difference between microelectrodes. A cell can be positioned by the pDEP force. Furthermore, owing to the cell positioning hole on the microelectrode, a single cell can be positioned in the cell positioning hole to achieve single cell positioning with high accuracy and high sensitivity.

More than one cell positioning units described above can be arranged to form a cell positioning array.

In the present disclosure, the microelectrodes have a dimension in micron scale, such as about several microns to several tens of microns.

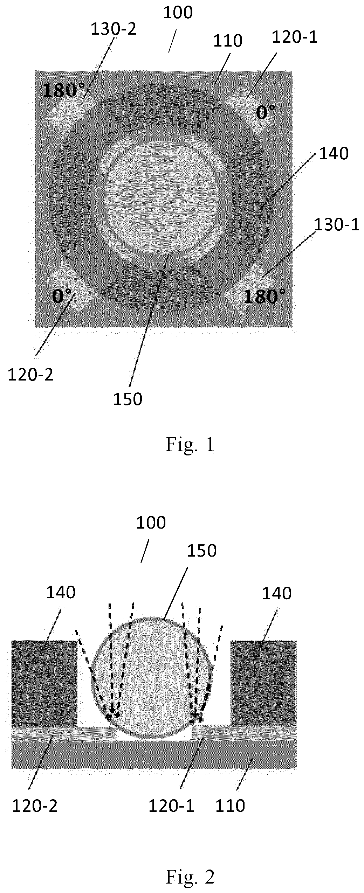

As illustrated in Figure 1, in the present embodiment, the microelectrodes having a stripe shape are disposed on a circumference at uniform or non-uniform internals with the tips of the microelectrodes being arranged towards an aggregating center. Thus, pDEP force fields are generated in an area where the microelectrode tips towards the center surround. Once voltage signals with phase difference are applied to the microelectrodes, strongest electric field will be generated in the area where cells can be positioned.

In a specific embodiment, two pairs of microelectrodes, i.e., four electrodes, are formed on the substrate 110. The first microelectrode 120-1, the second microelectrode 120-2, the third microelectrode 130-1 and the fourth microelectrode 130-2 having a stripe shape are disposed on a circumference at uniform internals. Tips of each pair of microelectrodes 120-1 and 120-2 are disposed oppositely with a distance therebetween smaller than the dimension of the cell to be detected to get better cell positioning. In the specific embodiment, two pairs of microelectrodes are disposed symmetrically. However it is illustrative only. The quantity and arrangement of the microelectrodes can be disposed as appropriate in other specific embodiments.

In the present embodiment, adjacent microelectrodes are arranged at intervals with voltage signals of opposite phase being applied. As illustrated in Figure 1, the opposite first microelectrode 120-1 and second microelectrode 120-2, and the opposite third microelectrode 130-1 and 130-2 are applied the same AC voltage signals, while the AC voltage signals applied to two adjacent microelectrodes have the same amplitude, frequency and opposite phase. In the specific embodiment, the voltage signals having opposite phases, i.e., the phase difference is 180°, are applied to adjacent microelectrodes. However, it is illustrative only. In other specific embodiments, voltage signals may be applied to microelectrodes in other configurations, as long as there exists a phase difference between voltage signals of a microelectrode and any other microelectrode so as to provide AC voltage signals, such that strong electric fields can be generated in the edge of the microelectrode tips for cell positioning.

In the present embodiment, a strongest electric field may be generated in the edge of the microelectrode tips once the AC voltage signals are applied to the microelectrodes. Cells will be attracted to move to edge of the tips by the pDEP force and finally be positioned thereto.

Furthermore, a cell positioning hole 140 is formed on the microelectrodes in the area for positioning cells. The cell positioning hole has an accommodation space for accommodating a single cell only to restrict a single cell into the cell positioning hole. In this embodiment, the cell positioning hole is located above the area defined by opposite microelectrode tips, which are exposed in the accommodation space of the cell positioning hole 140. More specifically, the cell positioning hole has a circular shape and has a diameter of about 1-2 times the cell diameters of the cell 150, such that only a single cell is positioned into the positioning hole by attraction of the DEP force to achieve single cell positioning. In the embodiment, the cell positioning hole has a circular shape. However, the shape of the positioning hole is not limited thereto, and may be a square, polygon or any other shape with a continuous or discontinuous perimeter. In case of a square shape, it may have a side which length is larger than the diameter and smaller than two times the diameter of the cell 150.

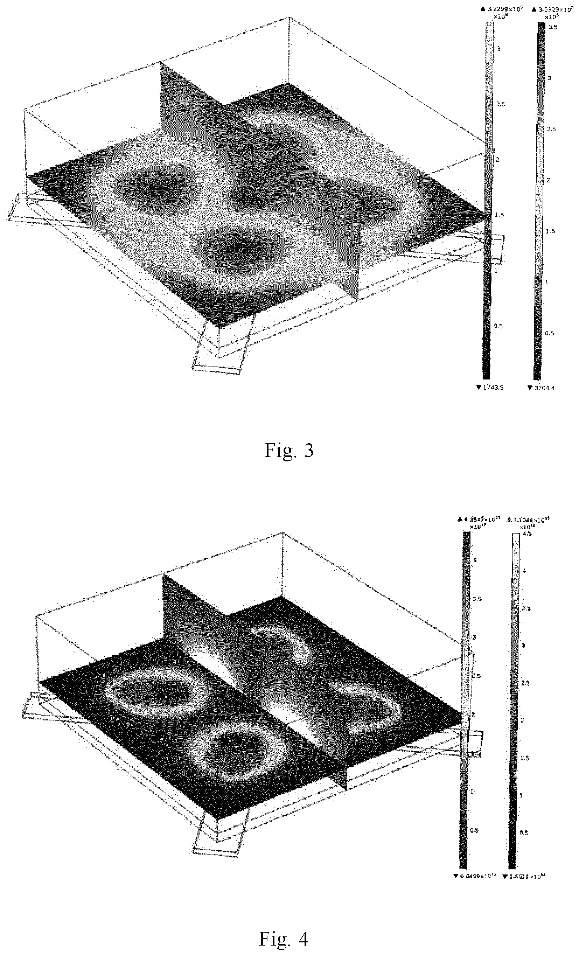

A three-dimensional finite element model is constructed for the cell positioning unit described above. In particular, the microelectrode has a width of about 4 microns and a thickness of about 100 microns. The distance of adjacent microelectrodes is 5 microns, and the distance of opposite microelectrodes is 15 microns. The amplitude of the applied AC voltage signal is 2V. Then a simulation analysis is made on the cell positioning unit by means of a finite element numerical simulation software named COMSOL. Figure 3, Figure 4 schematically illustrate microelectrode electric field distribution (E) and DEP force factor (∇| E|²) according to modeling for the cell positioning unit, respectively. As shown in Figure 3 and Figure 4, the electric field intensity has the strongest value at the edge on the surface of opposite microelectrode tips, and the DEP force factor has a highest value as well. Thus, the positioning area for positioning a cell is formed at tips of four opposite microelectrodes to position a cell to the area by pDEP force. In addition, a cell positioning hole can also be disposed in this area to contribute to singe cell positioning.

The cell positioning unit according to the present embodiment has been described above. The cell positioning unit can be disposed in an array to form a cell positioning array.

A cell positioning unit or array according to the present embodiment may be formed by the method as follows.

Firstly, a substrate 110 is provided, as illustrated in Figure 1.

The substrate may be an insulating base for supporting, for example, a silicon substrate with a dielectric layer thereon.

Then, microelectrode 120 and microelectrode 130 are formed, as illustrated in Figure 1.

The microelectrodes may be formed by depositing a metal layer and then patterning it. As shown in Figure 1, the microelectrodes have a stripe shape in an embodiment. Two pairs of microelectrodes are disposed oppositely. The microelectrodes have a width of about 4 microns and a thickness of about 100 microns. The distance of adjacent microelectrodes is about 5 microns, and the distance of opposite microelectrodes is about 15 microns.

Then, a cell positioning hole 140 is formed on the microelectrodes, as illustrated in Figure 1.

The cell positioning hole is formed of insulation materials, for example, in the present embodiment, by means of spin coating a thick photoresist layer and then etching by lithography. Opposite tips of the microelectrodes are exposed in the accommodation space of the cell positioning hole in which a single cell can be accommodated therein.

In other embodiments, the cell positioning hole can be formed by polymer, for example, Polydimethylsiloxane (PDMS). The cell positioning hole can be formed by conventional soft lithography process, and then can be bonded to the substrate of the microelectrode, so that the cell positioning hole can be manufactured on the microelectrodes.

Thus, a cell positioning unit or array according to the present embodiment is finally formed.

### Second Embodiment

In the present embodiment, the microelectrodes have a annular shape, the microelectrodes in a annular shape are concentrically arranged, and the strongest electric field intensity is generated in an area defined by the annular microelectrodes where the cell may be positioned. The cell positioning unit comprises: a substrate; annular microelectrodes on the substrate which are a pair of microelectrodes concentrically arranged, wherein the voltage signals applied to the pair of annular microelectrodes have phase difference; and a cell positioning hole on the microelectrodes, wherein the cell positioning hole has an accommodation space which exposes the microelectrodes and accommodates a single cell.

In a specific embodiment, a pair of annular microelectrodes 220-1 and 220-2 are concentrically arranged on the substrate 210. The inner ring microelectrode 220-1 has a diameter of about 2-50 microns and a width of about 2-20 microns, the outer ring microelectrode 220-2 has a diameter of about 5-100 microns and a width of about 2-20 microns, and the distance between the inner and outer microelectrodes is about 2-20 microns. The leading-out terminal of the inner microelectrode 220-1 is disposed above the outer microelectrode 220-2 by an in insulating layer 230 formed therebetween.

As in the first embodiment, there is a cell positioning hole (not shown in the drawing) formed on the microelectrodes. The cell positioning hole and the microelectrodes are concentrically disposed, and the inner microelectrode and the outer microelectrode are exposed in the accommodation space of the cell positioning hole, so that only a single cell can be attracted by the DEP force to be positioned into the positioning hole so as to achieve single cell positioning.

The cell positioning unit according to the present embodiment has been described above. The cell positioning unit can be arranged in an array to form a cell positioning array.

A method for forming the cell positioning unit or array according to the embodiment may comprise the following steps.

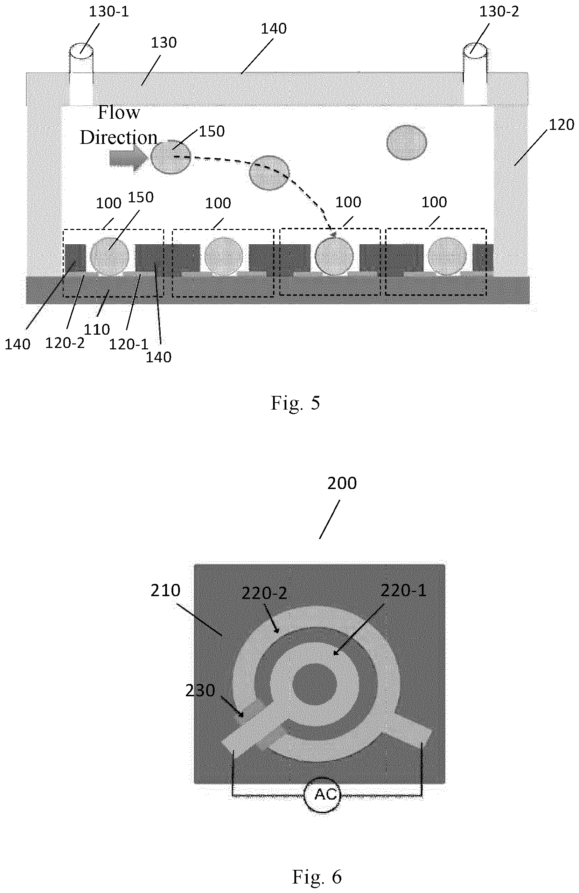

Firstly, a substrate 210 is provided, as shown in Figure 6.

The substrate may be an insulating base for supporting, for example, a silicon substrate with a dielectric layer thereon.

Then, microelectrode 220-1 and microelectrode 220-2 are formed, as illustrated in Figure 6.

The microelectrodes may be formed by depositing a metal layer and then patterning it. In the present embodiment, the microelectrodes are composed of annular microelectrodes 220-1 and 220-2 which are concentrically disposed. Then, an insulating layer 230 is formed on portions of the outer annular microelectrode 220-2. Finally, a leading-out terminal of the inner annular microelectrode 220-1 is formed on the insulating layer 230.

Then, a cell positioning hole is formed on the microelectrodes to be concentrically disposed with the microelectrodes. The cell positioning hole has an accommodation space for accommodating a single cell only.

The cell positioning hole can be formed of insulation materials. In the present embodiment, the cell positioning hole can be formed by spin coating a thick photoresist layer and then etching by lithography. The tips of the microelectrodes are exposed in the accommodation space of the cell positioning hole for accommodating a single cell only.

In other embodiments, the cell positioning hole can be formed of polymer, for example, Polydimethylsiloxane (PDMS). The cell positioning hole can be formed by conventional soft lithography, and can be bonded to the substrate of the microelectrode, so that the cell positioning hole can be manufactured on the microelectrodes.

Thus, the cell positioning unit or array according to the present embodiment is manufactured.

The cell positioning unit, the cell positioning array and the method for manufacturing the same have been described above.

Furthermore, a cell positioning device with the above described cell positioning unit built in is provided in the present disclosure for cell positioning and further analysis. As shown in Figure 5, the cell positioning device comprises: a cell positioning unit 100, a fluidic chamber 120 on the substrate 110, and a microfluidic chip 130 in the opening of the fluidic chamber 120. The micro fluidic chip 130 includes an entrance 130-1, an exit 130-2 and a cover plate 140. A microfluidic chip generally comprises an opening pipe, one or more sample entrances and sample exits for transportation of liquid samples. The cover plate covers the microfluidic chip while exposing the entrances and exits. While biological samples containing cells flowing through the microfluidic chip for transportation, an AC voltage signal having a specific frequency and amplitude is applied to the microelectrodes, so that the cells will be attracted by DEP force and attached to the microelectrodes with the interaction of fluidic force and DEP force, as indicated by the dashed arrow indicated in Figure 5.

In addition, a method for manufacturing the cell positioning device is provided in the present disclosure.

After the cell positioning units or arrays are formed, they can be combined with the fluidic chambers and microfluidic chips to form cell positioning devices.

In the present disclosure, soft materials such as PDMS can be used to form fluidic chambers, microfluidic chips can be formed by soft lithography of PDMS, and the cover plate can be made of transparent materials such as glass. The cover plate is combined with the microfluidic chip and the fluidic chamber by an external pressure to form a closed fluidic chamber. The fluidic chamber is further attached or bonded to the substrate by external pressure, so that a closed fluidic chamber having a sandwich structure is formed for cell positioning.

Preferred embodiments of the disclosure have been described above. However, it is not intended to limit the disclosure.

Although the disclosure has been described with reference to preferred embodiments, those skilled in the art should understood that various changes, modification and alterations may be made to the embodiments without departing the spirit and scope of the appended claims after reading the disclosed methods and technical content described above. Therefore, any change, modification, alternation and equivalents thereof made to the embodiments according to the technical essentials without departing from the scope of the disclosure other exemplary embodiments will fall within the scope defined by the attached claims.

## Claims

1. A cell positioning unit, comprising:
a substrate;
at least a pair of microelectrodes on the substrate, wherein the microelectrodes are disposed at intervals on a circumference such that pDEP forces are generated in an area where tips of the microelectrodes aggregate, and wherein a voltage signal applied to at least one microelectrode has a phase difference with a voltage signal applied to another microelectrode; and
a cell positioning hole on the microelectrodes, wherein the cell positioning hole has an accommodation space which exposes the pDEP force fields and accommodates a single cell.

2. The cell positioning unit of claim 1, wherein the microelectrodes have a stripe shape, and the tips of each pair of microelectrodes are disposed oppositely and exposed in the accommodation space of the cell positioning hole.

3. The cell positioning unit of claim 1, wherein the cell positioning hole has a circular or square shape, and has a diameter or side length of about 1-2 times the diameters of the cell to be detected.

4. A cell positioning unit, comprising:
a substrate;
a pair of annular microelectrodes on the substrate, wherein the pair of microelectrodes are concentrically disposed, and the voltage signals applied to the pair of microelectrodes have a phase difference therebetween; and
a cell positioning hole on the microelectrodes, wherein the cell positioning hole has an accommodation space which exposes the annular microelectrodes and accommodates a single cell.

5. The cell positioning unit of claim 4, wherein the cell positioning hole and the annular microelectrodes are concentrically disposed, and the cell positioning hole has a circular or square shape and has a diameter or side length of about 1-2 times the diameter of the cell to be detected.

6. A cell positioning array, comprising a plurality of cell positioning units of any one of claims 1-5 which are disposed in an array.

7. A cell positioning device, comprising:
the cell positioning array of claim 6;
a fluidic chamber on the substrate for accommodating the cell positioning array, wherein a microfluidic chip is disposed in an opening of the fluidic chamber.

8. A method for manufacturing a cell positioning unit/array, comprising:
providing a substrate;
forming at least a pair of microelectrodes on the substrate, wherein the microelectrodes are disposed at intervals on a circumference such that pDEP force fields are generated in an area where tips of the microelectrodes aggregate, and wherein a voltage signal applied to at least one microelectrode has a phase difference with a voltage signal applied to another microelectrode; and
forming a cell positioning hole on the microelectrodes, wherein the cell positioning hole has an accommodation space which exposes the pDEP force fields and accommodates a single cell.

9. The method of claim 8, wherein the microelectrodes have a stripe shape, and wherein tips of each pair of microelectrodes are disposed oppositely and exposed in the accommodation space of the cell positioning hole.

10. The method of claim 8, wherein the cell positioning hole has a circular or square shape, and has a diameter or side length of about 1-2 times the diameter of the cell to be detected.

11. A method for manufacturing a cell positioning unit/array, comprising:
providing a substrate;
forming at least a pair of annular microelectrodes on the substrate, wherein the pair of microelectrodes are concentrically disposed, and the voltage signals applied to the pair of microelectrodes have a phase difference therebetween; and
forming a cell positioning hole on the annular microelectrodes, wherein the cell positioning hole has an accommodation space which exposes the annular microelectrodes and accommodates a single cell.

12. A method of claim 11, wherein the cell positioning hole and the annular microelectrodes are concentrically disposed, and the cell positioning hole has a circular or square shape, and has a diameter or side length of about 1-2 times the diameter of the cell to be detected.

13. A method for manufacturing a cell positioning device, comprising:
forming a cell positioning array of any one of claims 8-12; and
forming a fluidic chamber on the substrate for accommodating the cell positioning array, wherein a microfluidic chip is disposed in an opening of the fluidic chamber.

14. The method for manufacturing a cell positioning device of claim 13, wherein the fluidic chamber is formed of polymer, and wherein one end of the fluidic chamber is bonded to the substrate while the other end is bonded to the microfluidic chip by an external pressure.
